# EUROPEAN PATENT APPLICATION

(11) **EP 4 473 847 A1**
(43) Date of publication of application: **11.12.2024**
(21) Application number: 23749870.4
(22) Date of filing: 03.02.2023
(51) Int. Cl.: A23L 33/135, A23K 10/18, A23L 5/00, A23L 33/15, A23L 33/155, A61K 9/20, A61K 35/744, A61K 47/26, A61K 47/38, A61P 1/00, A61P 3/06, A61P 37/04, C12N 1/20

(54) **LACTIC ACID BACTERIUM-CONTAINING TABLETS**

(30) Priority: 04.02.2022 JP 2022016450
(71) Applicant: KIRIN HOLDINGS KABUSHIKI KAISHA, Nakano-ku, Tokyo 164-0001 (JP)
(72) Inventor: TANAKA, Sayuri, Tokyo 164-0001 (JP); WATANABE, Junji, Tokyo 164-0001 (JP); KIMURA, Masao, Tokyo 164-0001 (JP); FUJIMORI, Yoshiyuki, Tokyo 164-0001 (JP); KANDA, Hirotatsu, Tokyo 164-0001 (JP); NAGASHIMA, Shinya, Tokyo 164-0001 (JP)
(74) Representative: Hoffmann Eitle
(86) International application number: PCT/JP2023/003619
(87) International publication number: WO 2023/149554

(57) **Abstract**

An object of the present invention is to provide a novel lactic acid bacterium-containing tablet. According to the present invention, there is provided a lactic acid bacterium-containing tablet comprising: (A) a lactic acid bacterium; (B) an excipient; and (C) a lubricant, wherein a content of the (C) in the tablet is less than 10 mass%, and a mass per tablet is 200 mg or more. The (B) preferably contains at least (B 1) hydroxypropyl cellulose and/or (B2) crystalline cellulose or β-cyclodextrin. The lactic acid bacterium is preferably a bacterium of the genus *Lactococcus.* According to the present invention, there is provided a lactic acid bacterium-containing tablet having high ingestion efficiency and excellent hardness.

## Description

### CROSS-REFERENCE TO RELATED APPLICATION

The present application enjoys the benefit of priority from the prior Japanese Patent Application No. 2022-016450 (filing date: February 4, 2022), the entire disclosures of which are incorporated herein by reference.

### TECHNICAL FIELD

The present invention relates to a lactic acid bacterium-containing tablet.

### BACKGROUND ART

Lactic acid bacteria are a generic term for Gram-positive bacteria that decompose sugars to produce lactic acid and acquire energy, and include bacteria of many genus species. Lactic acid bacteria have attracted attention as probiotics because they have effects such as cholesterol reduction and immunostimulation in addition to a role of adjusting the intestinal environment as intestinal resident bacteria of humans and animals.

As a food product containing probiotics, for example, an intestinal environment improving food product containing a colon disintegrable preparation containing a bifidobacterium and a lactic acid bacterium preparation (Patent Document 1) and a viable bacterium-containing preparation containing a beneficial intestinal bacterium (Patent Document 2) are known.

### Reference List

### Patent Documents

Patent Document 1: JP 2013-226095 A
Patent Document 2: JP 2018-158947 A

### SUMMARY OF THE INVENTION

### Technical Problem

An object of the present invention is to provide a lactic acid bacterium-containing tablet having high ingestion efficiency and excellent hardness.

### Solution to Problem

According to the present invention, the following inventions are provided.
[1] A lactic acid bacterium-containing tablet comprising: (A) a lactic acid bacterium; (B) an excipient; and (C) a lubricant, wherein a content of the (C) in the tablet is less than 10 mass%, wherein a mass per tablet is 200 mg or more.
[2] The lactic acid bacterium-containing tablet according to [1], wherein the (B) contains at least (B 1) hydroxypropyl cellulose.
[3] The lactic acid bacterium-containing tablet according to [1] or [2], wherein the (B) contains at least one or both of (B2) crystalline cellulose and β-cyclodextrin.
[4] The lactic acid bacterium-containing tablet according to [2] or [3], wherein a content of the (B 1) in the tablet is 1 to 20 mass%.
[5] The lactic acid bacterium-containing tablet according to any one of [2] to [4], wherein a ratio of the content (mass%) of the (B1) to the content (mass%) of the (C), expressed as (B1)/(C), is 0.5 to 6.
[6] The lactic acid bacterium-containing tablet according to any one of [1] to [5], wherein the (B) further contains maltose.
[7] The lactic acid bacterium-containing tablet according to any one of [1] to [6], wherein a ratio of a content (mass%) of the (A) to the content (mass%) of the (C), expressed as (A)/(C), is 0.5 to 7.5.
[8] The lactic acid bacterium-containing tablet according to any one of [1] to [7], wherein the content of the (A) in the tablet is 5 to 30 mass%.
[9] The lactic acid bacterium-containing tablet according to any one of [1] to [8], wherein the (A) is a lactic acid bacterium-sterilized dry powder.
[10] The lactic acid bacterium-containing tablet according to any one of [1] to [9], wherein the lactic acid bacterium of the (A) is a bacterium of the genus *Lactococcus.*
[11] The lactic acid bacterium-containing tablet according to [10], wherein the bacterium of the genus *Lactococcus* is *Lactococcus lactis* subsp. *lactis* JCM5805.
[12] The lactic acid bacterium-containing tablet according to any one of [1] to [11], wherein the (C) is a fatty acid ester.
[13] The lactic acid bacterium-containing tablet according to any one of [1] to [12], further comprising one or more selected from the group consisting of vitamin, 3-hydroxyisovaleric acid, 3-hydroxyisovaleric acid calcium, ornithine, and ornithine hydrochloride.
[14] The lactic acid bacterium-containing tablet according to any one of [1] to [13], which is a food product.
[15] The lactic acid bacterium-containing tablet according to any one of [1] to [14], wherein a hardness of the tablet is 50 N to 200 N.
[16] The lactic acid bacterium-containing tablet according to any one of [1] to [15], wherein the vitamin is L-ascorbic acid-2-glucoside.
[17] The lactic acid bacterium-containing tablet according to any one of [1] to [16], wherein the content of the (C) in the tablet is 3 mass% or more.

According to the present invention, it is possible to provide a lactic acid bacterium-containing tablet having high ingestion efficiency and excellent hardness. Therefore, the tablet of the present invention is advantageous in that a person in need thereof can take the tablet on a daily and continuous basis without an ingestion burden.

### BRIEF DESCRIPTION OF THE DRAWING

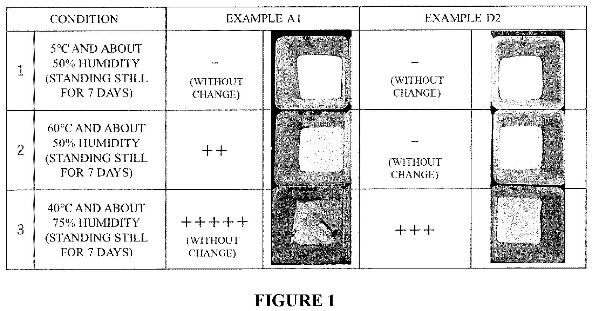

Fig. 1 is a view showing evaluation results of the degree of browning over time of Example A1 and Example D2.

### DETAILED DESCRIPTION OF THE INVENTION

### <<Lactic Acid Bacterium-Containing Tablet>>

### <Lactic Acid Bacterium>

A lactic acid bacterium contained in a lactic acid bacterium-containing tablet of the present invention (sometimes referred to simply as "tablet" in the present specification) is not particularly limited, spherical-shaped lactic acid bacteria are exemplified, and examples thereof include lactic acid bacteria of the genera *Lactococcus, Leuconostoc, Pediococcus, Streptococcus, Enterococcus,* and *Lactobacillus.* The lactic acid bacterium contained in the tablet of the present invention is preferably a lactic acid bacterium of the genus *Lactococcus* or *Pediococcus.*

Examples of the bacterium of the genus *Lactococcus* include *Lactococcus lactis, Lactococcus lactis* subsp. *lactis, Lactococcus garvieae, Lactococcus lactis* subsp. *cremoris,* and *Lactococcus lactis* subsp. *hordniae,* and *Lactococcus lactis* subsp. *lactis* is preferable.

Specific examples of the bacterium of the genus *Lactococcus* include *Lactococcus lactis* subsp. *lactis* JCM 5805, *Lactococcus lactis* subsp. *lactis* NBRC 12007, *Lactococcus lactis* subsp. *lactis* NRIC 1150, *Lactococcus lactis* subsp. *lactis* JCM 20101, *Lactococcus lactis* subsp. *lactis* JCM 7638, *Lactococcus lactis* subsp. *lactis* ATCC 11454, *Lactococcus garvieae* NBRC 100934, *Lactococcus lactis* subsp. *cremoris* JCM 16167, *Lactococcus lactis* subsp. *cremoris* NBRC 100676, *Lactococcus lactis* subsp. *hordniae* JCM 1180, and *Lactococcus lactis* subsp. *hordniae* JCM 11040, and *Lactococcus lactis* subsp. *lactis* JCM 5805 is preferable.

Examples of bacteria of the genus *Leuconostoc* include *Leuconostoc lactis.* Specific examples of the bacteria of the genus *Leuconostoc* include *Leuconostoc lactis* NBRC 12455.

Examples of bacteria of the genus *Pediococcus* include *Pediococcus acidilactici, Pediococcus pentosaceus, Pediococcus cellicola, Pediococcus claussenii, Pediococcus damnosus, Pediococcus ethanolidurans, Pediococcus inopinatus, Pediococcus parvulus,* and *Pediococcus stilesii.* Specific examples of the bacteria of the genus *Pediococcus* include *Pediococcus acidilactici* JCM 8797, *Pediococcus damnosus* JCM 5886, and *Pediococcus acidilactici* K15.

Examples of bacteria of the genus *Streptococcus* include *Streptococcus thermophilus.*

Examples of bacteria of the genus *Enterococcus* include *Enterococcus faecalis.*

Examples of bacteria of the genus *Lactobacillus* include *Lactobacillus paracasei, Lactobacillus delbrueckii, Lactobacillus acidophilus, Lactobacillus casei, Lactobacillus fructivorans, Lactobacillus hilgardii, Lactobacillus rhamnosus, Lactobacillus plantarum, Lactobacillus gasseri,* and *Lactobacillus acidophilus.* Specific examples of the bacteria of the genus *Lactobacillus* include *Lactobacillus paracasei* KW3110, *Lactobacillus paracasei* MCC1849, *Lactobacillus rhamnosus* GG, *Lactobacillus gasseri* SBT2055, *Lactobacillus plantarum* L-137, and *Lactobacillus acidophilus* L-92.

Among the above lactic acid bacterium strains, JCM strains are available from Japan Collection of Microorganisms of RIKEN BioResource Center (3-1-1, Koyadai, Tsukuba-shi, Ibaraki), NBRC strains are available from Biological Resource Center of the National Institute of Technology and Evaluation (2-5-8, Kazusa-kamatari, Kisarazu-shi, Chiba), NRIC strains are available from the Culture Collection Center, Tokyo University of Agriculture (1-1-1, Sakuragaoka, Setagaya, Tokyo), and ATCC strains are available from American type culture collection (USA). *Lactobacillus paracasei* KW3110 is deposited under Accession No. FERM BP-08634 (date of deposit: February 20, 2004) to the International Patent Organism Depositary, National Institute of Advanced Industrial Science and Technology (Chuo #6, 1-1-1, Higashi, Tsukuba-shi, Ibaraki 305-8566, Japan) (currently International Patent Organism Depositary, Biotechnology Center, National Institute of Technology and Evaluation (NITE-IPOD) (#120, 2-5-8, Kazusa-kamatari, Kisarazu-shi, Chiba 292-0818)), which is an international depositary authority under the Budapest Treaty on the International Recognition of the Deposit of Microorganisms for the Purposes of Patent Procedure. A derivative of *Lactobacillus paracasei* KW3110 is deposited under Accession No. FERM BP-08635 (date of deposit: February 20, 2004) to the same International Patent Organism Depositary.

*Lactococcus lactis* subsp. *lactis* JCM5805 is available from Japan Collection of Microorganisms of RIKEN BioResource Center as described above, but in the present invention, the same strain of the JCM5805 stored in a storage facility other than Japan Collection of Microorganisms of RIKEN BioResource Center can be used. Specifically, the same strain of the JCM5805 is available from Biological Resource Center of the National Institute of Technology and Evaluation (2-5-8, Kazusa-kamatari, Kisarazu-shi, Chiba), the Culture Collection Center, Tokyo University of Agriculture (1-1-1, Sakuragaoka, Setagaya, Tokyo), American type culture collection (USA), and the like.

As the lactic acid bacterium contained in the tablet of the present invention, either or both of a viable bacterial cell and a dead bacterial cell may be used, either or both of a lactic acid bacterial cell and a treated product thereof may be used, and the lactic acid bacterial cell may be in an isolated form or in a cultured form.

The lactic acid bacterium can be cultured by a known method using a known medium. As the medium, an MRS medium, a GAM medium, or an LM17 medium can be used, and a medium appropriately supplemented with an inorganic salt, a vitamin, an amino acid, an antibiotic substance, serum, or the like can be used. Culture can be carried out at 25 to 40°C for several hours to several days.

After culture, lactic acid bacterial cells can be collected by centrifugation or filtration. When dead lactic acid bacteria are used, the bacteria may be sterilized by heating in an autoclave or the like.

Examples of the treated product of the lactic acid bacterium include a crushed product of a viable bacterial cell or a dead bacterial cell (for example, ultrasonic crushed product), a dried product of a viable bacterial cell or a dead bacterial cell (for example, freeze-dried product), a crushed product of the dried product, and an enzyme-treated product of a viable bacterial cell or a dead bacterial cell. The dead bacterial cells can be obtained, for example, by heat treatment, treatment with drugs such as antibiotics, treatment with chemicals such as formalin, UV treatment, or radiation treatment such as treatment with γ rays. The enzyme-treated product also includes a treated product obtained by removing a cell wall from a lactic acid bacterium by an enzymatic or mechanical measure. A nucleic acid-containing fraction (for example, DNA or RNA) of the lactic acid bacterium is also included in the treated product of the lactic acid bacterium, and can be obtained by dissolving a lactic acid bacterial cell with a surfactant or the like and then precipitating the fraction with ethanol or the like. In the present invention, the "lactic acid bacterium" is used in the meaning including a viable bacterial cell and a dead bacterial cell of a lactic acid bacterial cell, a lactic acid bacterial cell and a treated product thereof, and lactic acid bacterial cells in an isolated form and in a cultured form.

In the present invention, as for the content of the lactic acid bacterium in one tablet, from the viewpoint of the hardness of the tablet, the lower limit value (a value of not less than or greater than) thereof can be, for example, 0.1 mass%, 1 mass%, 2 mass%, 3 mass%, 4 mass%, 5 mass%, 6 mass%, 7 mass%, 8 mass%, 9 mass%, or 10 mass%, and the upper limit value (a value of not more than or less than) thereof can be, for example, 50 mass%, 40 mass%, 35 mass%, 33 mass%, 31 mass%, 30 mass%, 28 mass%, 26 mass%, 25 mass%, 24 mass%, 22 mass%, 21 mass%, or 20 mass%. These lower limit values and upper limit values can be arbitrarily combined, the content of the lactic acid bacterium in one tablet can be, for example, 5 to 30 mass%, 10 to 30 mass%, 5 to 20 mass%, or 10 to 20 mass%.

In the present invention, as for the number of lactic acid bacteria in one tablet, from the viewpoint of the hardness of the tablet, the lower limit value (a value of not less than or greater than) thereof can be, for example, 1×10⁸ bacteria, 1×10⁹ bacteria, 1×10¹⁰ bacteria, 3×10¹⁰ bacteria, 5×10¹⁰ bacteria, or 1×10¹¹ bacteria, and the upper limit value thereof can be, for example, 1×10¹⁴ bacteria, 1×10¹³ bacteria, 1×10¹² bacteria, 5×10¹¹ bacteria, 4×10¹¹ bacteria, 3×10¹¹ bacteria, or 2×10¹¹ bacteria. These lower limit values and upper limit values can be arbitrarily combined, the number of lactic acid bacteria in one tablet can be, for example, 1×10⁸ to 1×10¹⁴ bacteria, 1×10⁹ to 1×10¹³ bacteria, 1×10¹⁰ to 1×10¹² bacteria, or 5×10¹⁰ to 3×10¹¹ bacteria. The number of lactic acid bacteria can be measured by, for example, a fluorescent staining method, flow cytometry, or a culture method, and is preferably measured by flow cytometry.

### <Excipient>

In the present invention, an excipient is used for handleability of the composition, improvement of molding, or the like. The excipient contained in the tablet of the present invention is not particularly limited, examples thereof include starches such as starch, pregelatinized starch, partially pregelatinized starch, and starch decomposition products, or derivatives thereof, dextrin, β-cyclodextrin, reduced palatinose, reduced maltose, sugar alcohols such as reduced maltose syrup, polysaccharides such as pullulan, disaccharides such as lactose and maltose, brewer's yeast, celluloses such as crystalline cellulose, low-substituted hydroxypropyl cellulose, hydroxypropyl cellulose, and hydroxypropyl methylcellulose, or derivatives thereof, purified sucrose, silicon dioxide (light anhydrous silicic acid), calcium silicate, titanium oxide, precipitated calcium carbonate, phosphate (tricalcium phosphate, calcium monohydrogen phosphate, calcium dihydrogen phosphate, or the like), and corn protein, and one kind or two or more kinds thereof may be used. As the excipient, commercially available products can be used. In the present invention, from the viewpoint of the hardness of the tablet, starch or a derivative thereof, cellulose or a derivative thereof, and saccharides such as sugar alcohol and a disaccharide are preferable, and either or both of hydroxypropyl cellulose and crystalline cellulose are more preferable. The tablet of the present invention can contain, as the excipient, at least one or both of (B 1) hydroxypropyl cellulose and (B2) crystalline cellulose, from the viewpoint of the hardness of the tablet. The tablet of the present invention can also contain at least (B 1) hydroxypropyl cellulose or at least one or both of (B2) crystalline cellulose and β-cyclodextrin, or can also contain (B 1) hydroxypropyl cellulose and at least one or both of (B2) crystalline cellulose and β-cyclodextrin, from the viewpoint of the hardness of the tablet.

In the present invention, as for the content of the excipient in one tablet, from the viewpoint of handleability of the composition, improvement of molding, or the like, the lower limit value (a value of not less than or greater than) thereof can be, for example, 3 mass%, 4 mass%, 5 mass%, 6 mass%, 7 mass%, 8 mass%, 9 mass%, 10 mass%, 20 mass%, 25 mass%, 30 mass%, 35 mass%, 40 mass%, 45 mass%, 50 mass%, or 55 mass%, and the upper limit value (a value of not more than or less than) thereof can be, for example, 90 mass%, 85 mass%, 80 mass%, 75 mass%, 74 mass%, 72 mass%, or 70 mass%. These lower limit values and upper limit values can be arbitrarily combined, and the content of the excipient in one tablet can be, for example, 10 to 90 mass%, 20 to 80 mass%, 30 to 70 mass%, 40 to 60 mass%, or 40 to 50 mass%.

In the present invention, as for the content of the (B 1) hydroxypropyl cellulose in one tablet, from the viewpoint of the hardness of the tablet, the lower limit value (a value of not less than or greater than) thereof can be, for example, 0.5 mass%, 1 mass%, 2 mass%, 3 mass%, or 3.5 mass%, and the upper limit value (a value of not more than or less than) thereof can be, for example, 30 mass%, 25 mass%, 23 mass%, 20 mass%, 18 mass%, 16 mass%, 15 mass%, 12 mass%, 10 mass%, 9 mass%, 8 mass%, or 7 mass%. These lower limit values and upper limit values can be arbitrarily combined, and the content of the (B 1) hydroxypropyl cellulose in one tablet can be, for example, 1 to 20 mass%, 1 to 10 mass%, 2 to 8 mass%, or 3 to 7 mass%.

In the present invention, as for the content of the (B2) crystalline cellulose or β-cyclodextrin in one tablet, from the viewpoint of the hardness of the tablet, the lower limit value (a value of not less than or greater than) thereof can be, for example, 0.5 mass%, 1 mass%, 2 mass%, 3 mass%, 4 mass%, 5 mass%, 10 mass%, 15 mass%, 20 mass%, 25 mass%, or 30 mass%, and the upper limit value (a value of not more than or less than) thereof can be, for example, 60 mass%, 55 mass%, 50 mass%, 45 mass%, or 40 mass%. These lower limit values and upper limit values can be arbitrarily combined, and the content of the (B2) crystalline cellulose or β-cyclodextrin in one tablet can be, for example, 10 to 50 mass%, 20 to 45 mass%, or 30 to 45 mass%.

### <Lubricant>

In the present invention, a lubricant is used, for example, to alleviate friction between a tableting machine punch and the tablet when a powder for tableting is compressed, and to prevent tableting failure such as sticking. The lubricant contained in the tablet of the present invention is not particularly limited as long as it is a component capable of achieving the above object, examples thereof include stearic acids such as stearic acid, calcium stearate, and magnesium stearate, or salts thereof, fatty acid esters such as sodium stearyl fumarate, polyethylene glycol, glycerin fatty acid ester, and sucrose fatty acid ester, vegetable fats and oils, hydrogenated oil, and talc, and one kind or two or more kinds thereof may be used. As the lubricant, commercially available products can be used. In the present invention, from the viewpoint of the hardness, stearic acid or a salt thereof, a fatty acid ester, and hydrogenated rapeseed oil are preferable, a fatty acid ester is more preferable, and sucrose fatty acid ester is still more preferable.

In the present invention, the content of the lubricant in one tablet is less than 10 mass% from the viewpoint of the tableting failure and the hardness of the tablet. As for the content of the lubricant in one tablet, the lower limit value (a value of not less than or greater than) thereof can be, for example, 0.5 mass%, 1 mass%, 1.5 mass%, 2 mass%, 2.5 mass%, 3 mass%, or 3.5 mass%, and the upper limit value (a value of not more than or less than) thereof can be, for example, 10 mass%, 9 mass%, 8 mass%, 7.5 mass%, 7 mass%, 6.5 mass%, or 6 mass%. These lower limit values and upper limit values can be arbitrarily combined, and the content of the lubricant in one tablet can be, for example, 1 to 9 mass%, 2 to 8 mass%, 3 to 6 mass%, or 3 to 10 mass%.

### <Vitamin>

The tablet of the present invention can further contain a vitamin in addition to the lactic acid bacterium, the excipient, and the lubricant. The vitamin contained in the tablet of the present invention is not particularly limited, examples thereof include coenzyme Q10, vitamin A, vitamin B1, vitamin B2, vitamin B6, vitamin B12, vitamin C, vitamin D (vitamin D3), vitamin E, nicotinic acid amide, calcium pantothenate, vitamin K2, folic acid, and pyrroloquinoline quinone, and one kind or two or more kinds thereof may be used. Examples of the vitamin C include L-ascorbic acid, sodium L-ascorbate, L-ascorbic acid stearate ester, L-ascorbic acid-2-glucoside, rapeseed oil-and-fat processed L-ascorbic acid, and corn starch processed L-ascorbic acid. Examples of the vitamin E include α, β, γ, and δ isomers of d-forms, which are natural products of tocopherol, dl-forms, which are synthetic products of tocopherol, and mixtures of one or more of these, and any of these can be used as tocopherol. As the vitamin, commercially available products can be used. In the present invention, it is preferable to contain a vitamin, more preferable to contain a plurality of kinds of vitamins, and still more preferable to contain either or both of L-ascorbic acid-2-glucoside and tocopherol or either or both of ascorbic acid and tocopherol.

In the present invention, as for the content of the vitamin in one tablet, from the viewpoint of the ingestion efficiency and the hardness of the tablet, the lower limit value (a value of not less than or greater than) thereof can be, for example, 3 mass%, 5 mass%, 10 mass%, 12 mass%, 13 mass%, 14 mass%, or 15 mass%, and the upper limit value (a value of not more than or less than) thereof can be, for example, 35 mass%, 30 mass%, 25 mass%, 24 mass%, 23 mass%, or 22 mass%. These lower limit values and upper limit values can be arbitrarily combined, and the content of the vitamin in one tablet can be, for example, 5 to 30 mass% or 10 to 25 mass%.

### <Other Formulation Components>

The tablet of the present invention may contain components other than those described above. Formulation components required for production of tablets are known, and can be appropriately selected and used, and examples thereof include fragrances, colorants, stabilizers, emulsifiers, disintegrants, and fluidity improvers.

### <Other Functional Component>

The tablet of the present invention may contain functional component other than the (A) lactic acid bacterium. The other functional component can be appropriately selected from known components and new components and used, and examples thereof include carotenoids, minerals, amino acids, amino acid derivatives, pharmaceutically active ingredients, plant extracts, and health food materials.

In the present invention, examples of the carotenoids include β-carotene, α-carotene, lutein, cryptoxanthin, zeaxanthin, lycopene, astaxanthin, and multi carotene.

In the present invention, examples of the minerals include calcium, magnesium, dolomite, manganese, zinc, iron, copper, selenium, chromium, sulfur, and iodine.

In the present invention, examples of the amino acids include aliphatic amino acids (specifically, glycine, alanine, and the like), branched-chain amino acids (specifically, valine, leucine, isoleucine, norleucine, and the like), hydroxyamino acids (specifically, serine, threonine, and the like), acidic amino acids (specifically, aspartic acid, glutamic acid, and the like), acidic amino acid amides (specifically, asparagine, glutamine, and the like), basic amino acids (specifically, lysine, hydroxylysine, arginine, ornithine, and the like), sulfur-comprising amino acids (specifically, cysteine, cystine, methionine, and the like), aromatic amino acids (specifically, phenylalanine, tyrosine, thyronine, and the like), heterocyclic amino acids (specifically, tryptophan, histidine, and the like), imino acids (specifically, proline, 4-hydroxyproline, and the like), salts thereof (for example, ornithine hydrochloride), and hydrates thereof.

In the present invention, examples of the amino acid derivatives include acetylglutamine, acetylcysteine, carboxymethylcysteine, acetyltyrosine, acetylhydroxyproline, 5-hydroxyproline, glutathione, creatine, S-adenylmethionine, glycylglycine, glycylglutamine, dopa, alanylglutamine, carnitine, γ-aminobutyric acid, 3-hydroxyisovaleric acid, salts thereof (for example, 3-hydroxyisovaleric acid calcium), and hydrates thereof.

In the present invention, examples of the pharmaceutically active ingredients include aspirin, acetaminophen, ethenzamide, ibuprofen, diphenhydramine, chlorpheniramine, dihydrocodeine, noscapine, methylephedrine, caffeine, serrapeptase, lysozyme, diclofenac sodium, ketoprofen, indometacin, bucolome, pentazocine, chlorpromazine, reserpine, alprazolam, chlordiazepoxide, diazepam, imipramine, maprotiline, estazolam, nitrazepam, diazepam, sodium phenobarbital, scopolamine, papaverine, citicoline, meclofenoxate, phenytoin, carbamazepine, isoproterenol, diastase, lansoprazole, omeprazole, rabeprazole, famotidine, cimetidine, ranitidine, dextromethorphan, guanfacine, codeine, difenidol, metoclopramide, levallorphan, theophylline, salbutamol, amlexanox, seratrodast, oxytetracycline, triamcinolone acetonide, chlorhexidine, lidocaine, diphenhydramine, promethazine, isothipendyl, digoxin, procainamide, propranolol, pindolol, isosorbide, furosemide, delapril, captopril, hydralazine, labetalol, manidipine, candesartan cilexetil, methyldopa, losartan, valsartan, eprosartan, irbesartan, tasosartan, telmisartan, forasartan, phenylephrine, carbocromen, molsidomine, verapamil, simvastatin, pravastatin, trepibutone, cefalexin, amoxicillin, pivmecillinam, cefotiam, cefozopran, cefinenoxime, cefsulodin sodium, ampicillin, ciclacillin, sulbenicillin sodium, nalidixic acid, enoxacin, carumonam sodium, tolbutamide, voglibose, pioglitazone, troglitazone, acarbose, miglitol, emiglitate, ipriflavone, methocarbamol, meclizine, dimenhydrinate, liothyronine sodium, dexamethasone, prednisolone, oxendolone, leuprorelin, opium, morphine, ipecacuanha, oxycodone, opium alkaloid, cocaine, allopurinol, colchicine, 5-fluorouracil, mitomycin, salts thereof, and hydrates thereof.

In the present invention, examples of the plant extracts include extracts of aloe, chlorella, prune, propolis, agaricus, *Panax ginseng,* ginkgo leaf, kale, *Litchi chinensis, Serenoa repens,* turmeric, curcumin, *Prunus mume* extract, grape seed, pine resin extract, germinated brown rice, shiitake mycelium extract, *Rubus suavissimus, Hydrangea serrata, Phellinus Linteus,* sesame, garlic, champignon, *Garcinia cambogia,* milk thistle extract, silymarin, St. John's Wort, mulberry leaf, *Gymnema sylvestre, Perillaftutescens, Plantago asiatica,* hardy rubber tree tee, oolong tea, *Sasa veitchii,* guava, *Panax notoginseng,* citrus, *Uncaria tomentosa,* Siberian ginseng, *Monascus purpureus,* maca, Cordyceps sinensis, camomile, chili pepper, and *Pueraria lobata* subsp. *lobata.* The method for producing a plant extract is not particularly limited, but includes raw material plants of plant extracts, processed products thereof, and the like. Examples of the processed products include products obtained by separating and concentrating a pulverized product of a fruit, seed, leaf, or the like of a plant or an extract extracted from a fruit, seed, leaf, or the like of a plant with an aqueous ethanol solution or the like.

In the present invention, examples of the health food materials include royal jelly, dietary fiber, proteins, bifidobacteria, chitosan, health vinegar, yeast, nucleic acid, glucosamine, lecithin, polyphenol, egg yolk oil, phytosterol, docosahexaenoic acid, cartilage of animals, fish, and shellfish, soft-shelled turtle, phosphatidylserine, lactoferrin, freshwater clams, eicosapentaenoic acid, germanium, enzymes, nattokinase, creatine, carnitine, citric acid, raspberry ketone, coenzyme Q10, methylsulfonylmethane, and soybean peptides bonded with phospholipids.

In the present invention, as for the content of the other functional component in one tablet, from the viewpoint of the hardness of the tablet, the lower limit value (a value of not less than or greater than) thereof can be, for example, 1 mass%, 10 mass%, 20 mass%, 30 mass%, 40 mass%, 50 mass%, 60 mass%, or 70 mass%, and the upper limit value (a value of not more than or less than) thereof can be, for example, 95 mass%, 90 mass%, or 80 mass%. These lower limit values and upper limit values can be arbitrarily combined, and the content of the other functional component in one tablet can be, for example, 40 to 95 mass%, 50 to 95 mass%, 60 to 95 mass%, or 70 to 90 mass%.

According to a preferred aspect of the present invention, there is provided a lactic acid bacterium-containing tablet comprising: (A) a lactic acid bacterium; (B) an excipient; (C) a lubricant; and (D) other functional component, wherein a content of the (C) in the tablet is less than 10 mass%, wherein a mass per tablet is 200 mg or more. When the other functional component is ornithine, ornithine hydrochloride, 3-hydroxyisovaleric acid, or 3-hydroxyisovaleric acid calcium, as for the content of each component in one lactic acid bacterium-containing tablet, the content of the (A) lactic acid bacterium can be preferably 1 to 30 mass% and more preferably 1 to 10 mass%, the content of the (B) excipient can be preferably 2 to 40 mass% and more preferably 2 to 20 mass%, the content of the (C) lubricant can be preferably 2 to 9 mass% and more preferably 2 to 8 mass%, and the content of the (D) other functional component can be preferably 60 to 95 mass% and more preferably 70 to 90 mass%. The lactic acid bacterium-containing tablet can contain, as the (B) excipient, (B1) hydroxypropyl cellulose and/or (B2) crystalline cellulose or β-cyclodextrin. In this case, as for the contents of (B1) and (B2) in one lactic acid bacterium-containing tablet, the content of the (B1) hydroxypropyl cellulose can be 2 to 8 mass%, preferably 3 to 7 mass%, and the content of the (B2) crystalline cellulose or β-cyclodextrin can be 0.5 to 20 mass%, preferably 1 to 15 mass%.

### <Content Ratio of Constituent>

In the tablet of the present invention, a ratio of the content (mass%) of the (B1) hydroxypropyl cellulose to the content (mass%) of the (C) lubricant in the tablet, expressed as (B1)/(C), can be set in a predetermined range. As for the ratio of the content (mass%) of the (B1) hydroxypropyl cellulose to the content (mass%) of the (C) lubricant in the tablet, expressed as (B1)/(C), from the viewpoint of fluidity at the time of tablet production of a mixed powder as a raw material of the tablet and the hardness of the tablet, the lower limit value (a value of not less than or greater than) thereof can be, for example, 0.5, 0.6, 0.7, 0.8, 0.9, or 1, and the upper limit value (a value of not more than or less than) thereof can be, for example, 6, 5.5, 5, 4.5, 4, or 3. These lower limit values and upper limit values can be arbitrarily combined, and the ratio of the content (mass%) of the (B1) hydroxypropyl cellulose to the content (mass%) of the (C) lubricant, expressed as (B1)/(C), can be, for example, 0.5 to 6, 0.8 to 5, or 0.8 to 3.

In the tablet of the present invention, a ratio of the content (mass%) of the (A) lactic acid bacterium to the content (mass%) of the (C) lubricant in the tablet, expressed as (A)/(C), can also be set in a predetermined range. As for the ratio of the content (mass%) of the (A) lactic acid bacterium to the content (mass%) of the (C) lubricant in the tablet, expressed as (A)/(C), from the viewpoint of the hardness of the tablet, the lower limit value (a value of not less than or greater than) thereof can be, for example, 0.5, 1, 1.5, 2, 2.5, or 3, and the upper limit value (a value of not more than or less than) thereof can be, for example, 8.5, 8, 7.5, 7, 6.5, or 6. These lower limit values and upper limit values can be arbitrarily combined, and the ratio of the content (mass%) of the (A) lactic acid bacterium to the content (mass%) of the (C) lubricant, expressed as (A)/(C), can be, for example, 0.5 to 7.5, 1 to 7.5, 2 to 7, or 3 to 6.

### <Tablet and Characteristics thereof>

The tablet of the present invention can be orally fed to humans and non-human animals, and a typical ingestion form thereof is a food product. In the present specification, the "food product" is used in the meaning including health food products, functional food products, nutritional supplements, foods with health claims (for example, foods for specified health uses, foods with nutrient function claims, and foods with function claims), foods for special dietary uses (for example, infant formulas, foods for pregnant or lactating women, and foods for patients), and supplements. When the tablet of the present invention is ingested by an animal other than a human, needless to say, the food product referred to in the present invention is used as a feed (including a pet food). The tablet of the present invention can also be orally administered to humans and non-human animals, and the administration form can also be a pharmaceutical product and a quasi-pharmaceutical product.

As shown in Examples below, the tablet of the present invention has high ingestion efficiency and excellent hardness. Here, the "ingestion efficiency" means that an ingestion amount per day or per one time can be efficiently ingested, and specifically, a predetermined ingestion amount can be ingested with a smaller number of times of ingestion and/or a smaller number of ingested tablets. The number of times of ingestion per day is preferably 3 times or less, more preferably 2 time or less, and still more preferably 1 time or less. The number of ingested tablets per day is preferably 6 or less, more preferably 4 or less, further preferably 2 or less, and still more preferably 1 or less. Therefore, the tablet of the present invention is advantageous in that a person in need thereof can take the tablet on a daily and continuous basis without an ingestion burden.

From the viewpoint of the ingestion efficiency, the mass per tablet of the present invention is preferably large, but from the viewpoint of prevention of tableting failure and the hardness of the tablet, the lower limit value (a value of not less than or greater than) thereof can be, for example, 200 mg, 250 mg, 255 mg, 260 mg, 270 mg, 280 mg, 290 mg, 300 mg, 305 mg, or 310 mg, and the upper limit value (a value of not more than or less than) thereof can be, for example, 500 mg, 450 mg, 400 mg, 390 mg, 380 mg, 370 mg, 360 mg, or 350 mg. These lower limit values and upper limit values can be arbitrarily combined, and the mass per tablet can be, for example, 200 to 500 mg, 260 to 500 mg, or 300 to 400 mg.

As for the hardness of the tablet of the present invention, from the viewpoint of prevention of tableting failure and quality retention as a tablet, the lower limit value (a value of not less than or greater than) thereof can be, for example, 50 N, 55 N, 60 N, 65 N, 70 N, 75 N, 80 N, 85 N, or 90 N, and the upper limit value (a value of not more than or less than) thereof can be, for example, 200 N, 190 N, 180 N, 170 N, 150 N, 140 N, 135 N, 130 N, 125 N, or 120 N. These lower limit values and upper limit values can be arbitrarily combined, and the tablet hardness can be, for example, 50 to 200 N, 50 to 190 N, 50 to 180 N, 80 to 130 N, or 90 to 120 N. The hardness of the tablet can be measured by a commercially available tablet breaking strength meter, for example, a digital hardness meter (KHT-40N, FUJIWARA SCIENTIFIC CO., LTD.).

As shown in Examples below, the tablet of the present invention is also advantageous in that it has excellent disintegratability. The disintegratability of the tablet of the present invention can be evaluated, for example, by a disintegration time in a disintegration test performed using an auxiliary disk with reference to the disintegration test method of The Japanese Pharmacopoeia Eighteenth Edition. As for the disintegration time of the tablet of the present invention, from the viewpoint of absorbability into a living body, the lower limit value (a value of not less than or greater than) thereof can be, for example, 10 minutes, 15 minutes, 20 minutes, 25 minutes, or 30 minutes, and the upper limit value (a value of not more than or less than) thereof can be, for example, 75 minutes, 70 minutes, 65 minutes, 60 minutes, 55 minutes, 53 minutes, 50 minutes, or 45 minutes. These lower limit values and upper limit values can be arbitrarily combined, and the disintegration time of the tablet can be, for example, 10 to 65 minutes, 20 minutes to 60 minutes, 25 to 50 minutes, or 30 to 45 minutes.

The form of the tablet of the present invention is not particularly limited as long as it is in the form of a tablet, and examples thereof include core tablets (uncoated tablets) and tablets coated with coating materials, such as sugar-coated tablets, gelatincoated tablets, and film-coated tablets, but core tablets (uncoated tablets) are preferable. The tablet of the present invention is also preferably a swallow tablet.

The shape of the tablet of the present invention can be specified by the shape of the cylindrical surface of the tablet, and examples thereof include a circle (circular tablet), an ellipse (heteromorphic tablet), an oval (heteromorphic tablet), and a quadrangle (heteromorphic tablet), but from the viewpoint of ingestion efficiency, prevention of tableting failure, and the hardness of the tablet, a circle or an ellipse is preferable. When the shape of the tablet of the present invention is circular, the diameter (ϕ) is preferably 7 to 13 mm, more preferably 8 to 12 mm, and still more preferably 8 to 11 mm.

### <<Production Method>>

The tablet of the present invention can be produced by mixing raw materials including a lactic acid bacterium, an excipient, and a lubricant, and then compressionmolding (tableting) the obtained mixture. The tableting method is not particularly limited, and examples thereof include a method of tableting with a hydraulic hand press, a single-punch tableting machine, a rotary tableting machine, or the like using a tableting die, upper and lower punches for tableting. The tableting can be performed such that a tablet to be obtained has an appropriate hardness and is adjusted so as not to cause tableting failure. The tableting pressure can be appropriately adjusted according to a tableting method, a device used for tableting, the size and mass of the tablet, contained components, and the like. For example, as for the tableting pressure when about 330 mg of a tablet is compressed per tablet using a tableting machine (ϕ9 mm punch) described in Examples below, from the viewpoint of the hardness, the lower limit value (a value of not less than or greater than) thereof can be, for example, 1 kN, 2 kN, 3 kN, 4 kN, or 5 kN, and the upper limit value (a value of not more than or less than) thereof can be, for example, 30 kN, 27 kN, 25 kN, 23 kN, or 20 kN. These lower limit values and upper limit values can be arbitrarily combined, and the tableting pressure can be, for example, 1 to 30 kN, 1 to 25 kN, 1 to 23 kN, 1 to 20 kN, or 5 to 20 kN.

### EXAMPLES

The present invention will be described more specifically based on the following examples; however, the present invention is not limited to these examples.

### <<Production and Evaluation of Tablet (1)>>

### (1) Preparation of Lactic Acid Bacterium-Sterilized Dry Powder

As a lactic acid bacterium, *Lactococcus lactis* subsp. *lactis* JCM5805 (produced by Kirin Holdings Company, Limited) was used. The lactic acid bacterium-sterilized dry powder was prepared by culturing bacterial cells, washing the bacterial cell liquid obtained by culturing, then heat-treating the bacterial cell liquid to sterilize the bacterial cells, and then spray-drying the heat-treated bacterial cell liquid. The obtained lactic acid bacterium-sterilized dry powder (lactic acid bacterium concentration: 3.3×10¹² lactic acid bacteria/g) was used in the production of tablets of Examples A1 to A10 and Reference Examples A1 to A8.

### (2) Hardness Test

The hardness (N) of each sample was measured using a digital hardness meter (KHT-40N, manufactured by FUJIWARA SCIENTIFIC CO., LTD.). The test was performed 10 times, and the average value thereof was calculated and shown as a measured value of hardness.

### (3) Disintegration Test

In the disintegration test, measurement was performed using an auxiliary disk by a disintegration tester (NT-20H, Toyama Sangyo Co., Ltd.) according to "Disintegration Test Method" of "The Japanese Pharmacopoeia Eighteenth Edition". Specifically, the disintegration time of each of six sample tablets was measured, and the maximum value thereof was shown as the disintegration time.

### (4) Production and Evaluation Result 1 of Tablet

Tablets of Examples A1 to A10 and Reference Examples A1 to A8 were produced, and manufacturability was evaluated. The obtained tablets were subjected to mass and diameter (ϕ) measurements, a hardness test, and a disintegration test.

### Example A1

A mixed powder for tableting was obtained by mixing 15 g of a lactic acid bacterium-sterilized dry powder, 4.6 g of hydroxypropyl cellulose (CELNY SSL-SFP, manufactured by Nippon Soda Co., Ltd., the same applies hereinafter), 39 g of crystalline cellulose (Ceolus UF-F702, manufactured by Asahi Kasei Corporation, the same applies hereinafter), 4 g of sucrose fatty acid ester (RYOTO Sugar Ester S-370F, manufactured by Mitsubishi Chemical Group Corporation, the same applies hereinafter), 13 g of ascorbic acid (Ascorbic acid, manufactured by DSM Japan K.K., the same applies hereinafter), 6 g of tocopherol (Riken Dry E Mix F-20S, manufactured by Riken Vitamin Co., Ltd., the same applies hereinafter), and 18.4 g of maltose (SUNMALT-S, manufactured by Hayashibara Co., Ltd., the same applies hereinafter), and allowing the mixture to pass through a sieve with a mesh size of 500 µm.

The obtained mixed powder was compressed into tablets using a fully automatic single-punch tableting machine (AUTOTAB-535, manufactured by ICHIHASHI SEIKI CO., LTD., the same applies hereinafter) at a tableting pressure of 15.5 kN to obtain tablets each having a diameter (ϕ) of 9 mm and a mass of 335.5 mg. The measurement was performed 10 times, and average values thereof were shown as the measured values of the diameter (ϕ) and the mass of the tablets (the same applies hereinafter). The hardness of the tablet was 95.2 N and the tablet was disintegrated within 37 minutes.

### Example A2

A mixed powder for tableting was obtained by mixing 5 g of a lactic acid bacterium-sterilized dry powder, 4.6 g of hydroxypropyl cellulose, 39 g of crystalline cellulose, 4 g of sucrose fatty acid ester, 13 g of ascorbic acid, 6 g of tocopherol, and 28.4 g of maltose, and allowing the mixture to pass through a sieve with a mesh size of 500 µm.

The obtained mixed powder was compressed into tablets using a fully automatic single-punch tableting machine at a tableting pressure of 6.1 kN to obtain tablets each having a diameter (ϕ) of 9 mm and a mass of 336.0 mg. The hardness of the tablet was 95.9 N and the tablet was disintegrated within 27 minutes.

### Example A3

A mixed powder for tableting was obtained by mixing 30 g of a lactic acid bacterium-sterilized dry powder, 4.6 g of hydroxypropyl cellulose, 39 g of crystalline cellulose, 4 g of sucrose fatty acid ester, 13 g of ascorbic acid, 6 g of tocopherol, and 3.4 g of maltose, and allowing the mixture to pass through a sieve with a mesh size of 500 µm.

The obtained mixed powder was compressed into tablets using a fully automatic single-punch tableting machine at a tableting pressure of 20 kN to obtain tablets each having a diameter (ϕ) of 9 mm and a mass of 345.3 mg. The hardness of the tablet was 55.4 N and the tablet was disintegrated within 45 minutes.

### Example A4

A mixed powder for tableting was obtained by mixing 20 g of a lactic acid bacterium-sterilized dry powder, 4.6 g of hydroxypropyl cellulose, 39 g of crystalline cellulose, 4 g of sucrose fatty acid ester, 13 g of ascorbic acid, 6 g of tocopherol, and 13.4 g of maltose, and allowing the mixture to pass through a sieve with a mesh size of 500 µm.

The obtained mixed powder was compressed into tablets using a fully automatic single-punch tableting machine at a tableting pressure of 23 kN to obtain tablets each having a diameter (ϕ) of 9 mm and a mass of 332.9 mg. The hardness of the tablet was 89.5 N and the tablet was disintegrated within 37 minutes.

### Example A5

A mixed powder for tableting was obtained by mixing 15 g of a lactic acid bacterium-sterilized dry powder, 20 g of hydroxypropyl cellulose, 39 g of crystalline cellulose, 4 g of sucrose fatty acid ester, 13 g of ascorbic acid, 6 g of tocopherol, and 3 g of maltose, and allowing the mixture to pass through a sieve with a mesh size of 500 µm.

The obtained mixed powder was compressed into tablets using a fully automatic single-punch tableting machine at a tableting pressure of 3 kN to obtain tablets each having a diameter (ϕ) of 9 mm and a mass of 331.4 mg. The hardness of the tablet was 93.6 N and the tablet was disintegrated within 65 minutes.

### Example A6

A mixed powder for tableting was obtained by mixing 15 g of a lactic acid bacterium-sterilized dry powder, 4.6 g of hydroxypropyl cellulose, 4 g of sucrose fatty acid ester, 13 g of ascorbic acid, 6 g of tocopherol, and 57.4 g of maltose, and allowing the mixture to pass through a sieve with a mesh size of 500 µm.

The obtained mixed powder was compressed into tablets using a fully automatic single-punch tableting machine at a tableting pressure of 22 kN to obtain tablets each having a diameter (ϕ) of 9 mm and a mass of 337.8 mg. The hardness of the tablet was 73.4 N and the tablet was disintegrated within 30 minutes.

### Example A7

A mixed powder for tableting was obtained by mixing 15 g of a lactic acid bacterium-sterilized dry powder, 10 g of hydroxypropyl cellulose, 39 g of crystalline cellulose, 4 g of sucrose fatty acid ester, 13 g of ascorbic acid, 6 g of tocopherol, and 13 g of maltose, and allowing the mixture to pass through a sieve with a mesh size of 500 µm.

The obtained mixed powder was compressed into tablets using a fully automatic single-punch tableting machine at a tableting pressure of 5.6 kN to obtain tablets each having a diameter (ϕ) of 9 mm and a mass of 331.4 mg. The hardness of the tablet was 106.3 N and the tablet was disintegrated within 40 minutes.

### Example A8

A mixed powder for tableting was obtained by mixing 15 g of a lactic acid bacterium-sterilized dry powder, 15 g of hydroxypropyl cellulose, 39 g of crystalline cellulose, 4 g of sucrose fatty acid ester, 13 g of ascorbic acid, 6 g of tocopherol, and 8 g of maltose, and allowing the mixture to pass through a sieve with a mesh size of 500 µm.

The obtained mixed powder was compressed into tablets using a fully automatic single-punch tableting machine at a tableting pressure of 4 kN to obtain tablets each having a diameter (ϕ) of 9 mm and a mass of 332.7 mg. The hardness of the tablet was 116.7 N and the tablet was disintegrated within 53 minutes.

### Example A9

A mixed powder for tableting was obtained by mixing 15 g of a lactic acid bacterium-sterilized dry powder, 4.6 g of hydroxypropyl cellulose, 39 g of crystalline cellulose, 8 g of sucrose fatty acid ester, 13 g of ascorbic acid, 6 g of tocopherol, and 14.4 g of maltose, and allowing the mixture to pass through a sieve with a mesh size of 500 µm.

The obtained mixed powder was compressed into tablets using a fully automatic single-punch tableting machine at a tableting pressure of 8.9 kN to obtain tablets each having a diameter (ϕ) of 9 mm and a mass of 332.6 mg. The hardness of the tablet was 95.5 N and the tablet was disintegrated within 51 minutes.

### Example A10

A mixed powder for tableting was obtained by mixing 15 g of a lactic acid bacterium-sterilized dry powder, 4.6 g of hydroxypropyl cellulose, 39 g of crystalline cellulose, 6 g of sucrose fatty acid ester, 13 g of ascorbic acid, 6 g of tocopherol, and 16.4 g of maltose, and allowing the mixture to pass through a sieve with a mesh size of 500 µm.

The obtained mixed powder was compressed into tablets using a fully automatic single-punch tableting machine at a tableting pressure of 7.5 kN to obtain tablets each having a diameter (ϕ) of 9 mm and a mass of 345 mg. The hardness of the tablet was 91.6 N and the tablet was disintegrated within 43 minutes.

### Reference Example A1

A mixed powder for tableting was obtained by mixing 15 g of a lactic acid bacterium-sterilized dry powder, 15 g of hydroxypropyl cellulose, 39 g of crystalline cellulose, 10 g of sucrose fatty acid ester, 13 g of ascorbic acid, 6 g of tocopherol, and 12.4 g of maltose, and allowing the mixture to pass through a sieve with a mesh size of 500 µm.

The obtained mixed powder was compressed into tablets using a fully automatic single-punch tableting machine at a tableting pressure of 15 kN, but could not be molded into tablets.

### Reference Example A2

A mixed powder for tableting was obtained by mixing 15 g of a lactic acid bacterium-sterilized dry powder, 4.6 g of hydroxypropyl cellulose, 39 g of crystalline cellulose, 13 g of ascorbic acid, 6 g of tocopherol, and 22.4 g of maltose, and allowing the mixture to pass through a sieve with a mesh size of 500 µm.

The obtained mixed powder was compressed into tablets using a fully automatic single-punch tableting machine at a tableting pressure of 8.2 kN, but could not be molded into tablets.

### Reference Example A3

A mixed powder for tableting was obtained by mixing 15 g of a lactic acid bacterium-sterilized dry powder, 39 g of crystalline cellulose, 4 g of sucrose fatty acid ester, 13 g of ascorbic acid, 6 g of tocopherol, and 23 g of maltose, and allowing the mixture to pass through a sieve with a mesh size of 500 µm.

The obtained mixed powder was compressed into tablets using a fully automatic single-punch tableting machine at a tableting pressure of 23.5 kN, but could not be molded into tablets.

### Reference Example A4

A mixed powder for tableting was obtained by mixing 15 g of a lactic acid bacterium-sterilized dry powder, 4.6 g of hydroxypropyl cellulose, 39 g of crystalline cellulose, 2 g of sucrose fatty acid ester, 13 g of ascorbic acid, 6 g of tocopherol, and 20.4 g of maltose, and allowing the mixture to pass through a sieve with a mesh size of 500 µm.

The obtained mixed powder was compressed into tablets using a fully automatic single-punch tableting machine at a tableting pressure of 6.2 kN, but could not be molded into tablets.

### Reference Example A5

A mixed powder for tableting was obtained by mixing 15 g of a lactic acid bacterium-sterilized dry powder, 43.6 g of crystalline cellulose, 4 g of sucrose fatty acid ester, 13 g of ascorbic acid, 6 g of tocopherol, and 18.4 g of maltose, and allowing the mixture to pass through a sieve with a mesh size of 500 µm.

The obtained mixed powder was compressed into tablets using a fully automatic single-punch tableting machine at a tableting pressure of 15.0 kN, but could not be molded into tablets.

### Reference Example A6

A mixed powder for tableting was obtained by mixing 15 g of a lactic acid bacterium-sterilized dry powder, 39 g of crystalline cellulose, 4 g of sucrose fatty acid ester, 13 g of ascorbic acid, 6 g of tocopherol, 4.6 g of reduced maltose syrup (AMALTY MR-50, manufactured by Mitsubishi Shoji Foodtech Co., Ltd.), and 18.4 g of maltose, and allowing the mixture to pass through a sieve with a mesh size of 500 µm.

The obtained mixed powder was compressed into tablets using a fully automatic single-punch tableting machine at a tableting pressure of 19.3 kN, but could not be molded into tablets.

### Reference Example A7

A mixed powder for tableting was obtained by mixing 15 g of a lactic acid bacterium-sterilized dry powder, 39 g of crystalline cellulose, 4 g of sucrose fatty acid ester, 13 g of ascorbic acid, 6 g of tocopherol, 4.6 g of lactose (Lactose granule, manufactured by FREUND CORPORATION), and 18.4 g of maltose, and allowing the mixture to pass through a sieve with a mesh size of 500 µm.

The obtained mixed powder was compressed into tablets using a fully automatic single-punch tableting machine at a tableting pressure of 18.2 kN, but could not be molded into tablets.

### Reference Example A8

A mixed powder for tableting was obtained by mixing 15 g of a lactic acid bacterium-sterilized dry powder, 39 g of crystalline cellulose, 4 g of sucrose fatty acid ester, 13 g of ascorbic acid, 6 g of tocopherol, 4.6 g of dextrin (Sandex #30, manufactured by Sanwa Starch Co., Ltd.), and 18.4 g of maltose, and allowing the mixture to pass through a sieve with a mesh size of 500 µm.

The obtained mixed powder was compressed into tablets using a fully automatic single-punch tableting machine at a tableting pressure of 18.7 kN, but could not be molded into tablets.

Formulations and results of the tablets were as shown in Tables 1 and 2. In the tables, "(A)/(C)" is a value obtained by dividing the content (mass%) of the (A) lactic acid bacterium by the content (mass%) of the (C) lubricant, and "(B1)/(C)" is a value obtained by dividing the content (mass%) of the (B 1) excipient by the content (mass%) of the (C) lubricant (the same applies hereinafter). "-" in the tables indicates that a tablet could not be produced and thus measurement could not be performed (unmeasurable).

**[Table 1]**

| Table 1. Formulations and evaluation results of tablets | | | | | | | | | | | | |
|---|---|---|---|---|---|---|---|---|---|---|---|---|
| | | Component | Example | | | | | | | | Reference Example | |
| | | | A1 | A2 | A3 | A4 | A5 | A6 | A7 | A8 | A1 | A2 |
| (A) | Lactic acid bacterium | Lactic acid bacterium-sterilized dry powder (mass%) | 15 | 5 | 30 | 20 | 15 | 15 | 15 | 15 | 15 | 15 |
| (B1) | Excipient | Hydroxypropyl cellulose (mass%) | 4.6 | 4.6 | 4.6 | 4.6 | 20 | 4.6 | 10 | 15 | 4.6 | 46 |
| (B2) | Excipient | Crystalline cellulose (mass%) | 39 | 39 | 39 | 39 | 39 | 0 | 39 | 39 | 39 | 39 |
| (C) | Lubricant | Sucrose fatty acid ester (mass%) | 4 | 4 | 4 | 4 | 4 | 4 | 4 | 4 | 10 | 0 |
| | Vitamin | Ascorbic acid (mass%) | 13 | 13 | 13 | 13 | 13 | 13 | 13 | 13 | 13 | 13 |
| | Vitamin | Tocopherol (mass%) | 6 | 6 | 6 | 6 | 6 | 6 | 6 | 6 | 6 | 6 |
| (B) | Excipient | Maltose (mass%) balance | 18.4 | 28.4 | 3.4 | 13.4 | 3 | 57.4 | 13 | 8 | 12.4 | 22.4 |
| Total (mass%) | | | 100 | 100 | 100 | 100 | 100 | 100 | 100 | 100 | 100 | 100 |
| (A)/(C) | | | 3.75 | 1.25 | 7.5 | 5 | 3.75 | 3.75 | 3.75 | 3.75 | 1.5 | 0 |
| (B1)/(C) | | | 1.15 | 1.15 | 1.15 | 1.15 | 5 | 1.15 | 2.5 | 3.75 | 0.46 | 0 |
| | | Tableting pressure (kN) | 15.5 | 6.1 | 20 | 23 | 3 | 22 | 5.6 | 4 | 15 | 8.2 |
| Evaluation result | | Tableting failure | Absent | Absent | Absent | Absent | Absent | Absent | Absent | Absent | Present | Present |
| | | Diameter (ϕ) (mm) | 9 | 9 | 9 | 9 | 9 | 9 | 9 | 9 | - | - |
| | | Mass (mg) per tablet | 335.5 | 336 | 345.3 | 332.9 | 331.4 | 337.8 | 331.4 | 332.7 | - | - |
| | | Tablet hardness (N) | 95.2 | 95.9 | 55.4 | 89.5 | 93.6 | 73.4 | 106.3 | 116.7 | - | - |
| | | Disintegration time (within minutes) | 37 | 27 | 45 | 37 | 65 | 30 | 40 | 53 | - | - |

**[Table 2]**

| Table 2. Formulations and evaluation results of tablets | | | | | | | | | | |
|---|---|---|---|---|---|---|---|---|---|---|
| | | Component | Example | | Reference Example | | | | | |
| | | | A9 | A10 | A3 | A4 | A5 | A6 | A7 | A8 |
| (A) | Lactic acid bacterium | Lactic acid bacterium-sterilized dry powder (mass%) | 15 | 15 | 15 | 15 | 15 | 15 | 15 | 15 |
| (B1) | Excipient | Hydroxypropyl cellulose (mass%) | 4.6 | 4.6 | 0 | 4.6 | 0 | 0 | 0 | 0 |
| (B1) | Excipient | Reduced maltose syrup (mass%) | 0 | 0 | 0 | 0 | 0 | 4.6 | 0 | 0 |
| (B1) | Excipient | Lactose (mass%) | 0 | 0 | 0 | 0 | 0 | 0 | 4.6 | 0 |
| (B1) | Excipient | Dextrin (mass%) | 0 | 0 | 0 | 0 | 0 | 0 | 0 | 4.6 |
| (B2) | Excipient | Crystalline cellulose (mass%) | 39 | 39 | 39 | 39 | 436 | 39 | 39 | 39 |
| (C) | Lubricant | Sucrose fatty acid ester (mass%) | 8 | 6 | 4 | 2 | 4 | 4 | 4 | 4 |
| | Vitamin | Ascorbic acid (mass%) | 13 | 13 | 13 | 13 | 13 | 13 | 13 | 13 |
| | Vitamin | Tocopherol (mass%) | 6 | 6 | 6 | 6 | 6 | 6 | 6 | 6 |
| (B) | Excipient | Maltose (mass%) balance | 14.4 | 16.4 | 23 | 20.4 | 18.4 | 18.4 | 18.4 | 18.4 |
| Total (mass%) | | | 100 | 100 | 100 | 100 | 100 | 100 | 100 | 100 |
| (A)/(C) | | | 1.875 | 2.5 | 3.75 | 7.5 | 3.75 | 3.75 | 3.75 | 3.75 |
| (B1)/(C) | | | 0.58 | 0.77 | 0 | 2.3 | 0 | 1.15 | 1.15 | 1.15 |
| | | Tableting pressure (kN) | 8.9 | 7.5 | 23.5 | 6.2 | 150 | 19.3 | 18.2 | 18.7 |
| Evaluation result | | Tableting failure | Absent | Absent | Present | Present | Present | Present | Present | Present |
| | | Diameter (ϕ) (mm) | 9 | 9 | - | - | - | - | - | - |
| | | Mass (mg) per tablet (n = 10) | 332.6 | 345 | - | - | - | - | - | - |
| | | Tablet hardness (N) (n = 10) | 95.2 | 95.9 | - | - | - | - | - | - |
| | | Disintegration time (within minutes) | 51 | 43 | - | - | - | - | - | - |

From the results of Tables 1 and 2, it was confirmed that tableting cannot be performed in Reference Examples A1, A3, and A5 to A7 due to tableting failure (capping) and in Reference Examples 2 and 4 due to tableting failure (sticking), whereas tableting can be performed in Examples A1 to A10 without tableting failure. Since the tablets of Examples A1 to A10 contain about 3×10¹⁰ to about 4×10¹¹ lactic acid bacteria per tablet, the tablets have high ingestion efficiency and favorable hardness, so that the tablets are advantageous in that both ingestion efficiency and favorable hardness can be achieved. Since the tablets of Examples A1 to A10 have a disintegration time within 27 minutes to 65 minutes, the tablets are also advantageous in that absorbability into a living body is high.

### <<Production and Evaluation of Tablet (2)>>

For Examples B1 to B4 and C1, tablets were produced and evaluated by the same methods as described in "Production and Evaluation of Tablet (1)" described above, which include (1) Preparation of Lactic Acid Bacterium-Sterilized Dry Powder, (2) Hardness Test, (3) Disintegration Test, and (4) Production and Evaluation Result of Tablet. The disintegration test was performed on the tablet of Example C1.

### Example B1

A mixed powder for tableting was obtained by granulating, using a fluidized bed granulator, and mixing 2.8 g of a lactic acid bacterium-sterilized dry powder, 3.5 g of hydroxypropyl cellulose, 1.6 g of crystalline cellulose, 85.8 g of 3-hydroxyisovaleric acid calcium (KOBAYASHI HMBCa, manufactured by KOBAYASHI PERFUMERY CO., LTD., the same applies hereinafter), 5.3 g of calcium stearate (Calcium stearate, manufactured by Taihei Chemical Industrial Co., Ltd., the same applies hereinafter), 0.3 g of corn protein (Zein SSW, manufactured by SHINWA ALCOHOL INDUSTRY CO., LTD., the same applies hereinafter), 0.7 g of silicon dioxide (SYLOPAGE720, manufactured by FUJI SILYSIA CHEMICAL LTD., the same applies hereinafter), and 0.1 g of vitamin D (Dry Vitamin D3, manufactured by Mitsubishi Chemical Group Corporation, the same applies hereinafter), and then allowing the mixture to pass through a sieve with a mesh size of 500 µm.

The obtained mixed powder was compressed into tablets using a rotary tableting machine at a tableting pressure of 6 kN to obtain tablets each having a diameter (ϕ) of 9 mm and a mass of 298.8 mg. The hardness of the tablet was 139.1 N.

### Example B2

A mixed powder for tableting was obtained by granulating, using a fluidized bed granulator, and mixing 2.8 g of a lactic acid bacterium-sterilized dry powder, 3.5 g of hydroxypropyl cellulose, 1.6 g of crystalline cellulose, 85.8 g of 3-hydroxyisovaleric acid calcium, 5.3 g of calcium stearate, 0.3 g of corn protein, 0.7 g of silicon dioxide, and 0.1 g of vitamin D, and then allowing the mixture to pass through a sieve with a mesh size of 500 µm.

The obtained mixed powder was compressed into tablets using a rotary tableting machine at a tableting pressure of 8 kN to obtain tablets each having a diameter (ϕ) of 9 mm and 299.23 mg. The hardness of the tablet was 170.1 N.

### Example B3

A mixed powder for tableting was obtained by granulating, using a fluidized bed granulator, and mixing 2.8 g of a lactic acid bacterium-sterilized dry powder, 3.5 g of hydroxypropyl cellulose, 1.6 g of crystalline cellulose, 85.8 g of 3-hydroxyisovaleric acid calcium, 5.3 g of calcium stearate, 0.3 g of corn protein, 0.7 g of silicon dioxide, and 0.1 g of vitamin D, and then allowing the mixture to pass through a sieve with a mesh size of 500 µm.

The obtained mixed powder was compressed into tablets using a rotary tableting machine at a tableting pressure of 10 kN to obtain tablets each having a diameter (ϕ) of 9 mm and a mass of 299.29 mg. The hardness of the tablet was 170 N.

### Example B4

A mixed powder for tableting was obtained by granulating, using a fluidized bed granulator, and mixing 2.8 g of a lactic acid bacterium-sterilized dry powder, 3.5 g of hydroxypropyl cellulose, 1.6 g of crystalline cellulose, 85.8 g of 3-hydroxyisovaleric acid calcium, 5.3 g of calcium stearate, 0.3 g of corn protein, 0.7 g of silicon dioxide, and 0.1 g of vitamin D, and then allowing the mixture to pass through a sieve with a mesh size of 500 µm.

The obtained mixed powder was compressed into tablets using a rotary tableting machine at a tableting pressure of 5 kN to obtain tablets each having a diameter (ϕ) of 9 mm and a mass of 299.29 mg. The hardness of the tablet was 110.6 N.

### Example C1

A mixed powder for tableting was obtained by mixing 3.34 g of a lactic acid bacterium-sterilized dry powder, 10 g of β-cyclodextrin (Celldex B100, manufactured by NIHON SHOKUHIN KAKO CO., LTD.), 75 g of ornithine hydrochloride (L-ornithine hydrochloride KYOWA (pulverized product), manufactured by KYOWA HAKKO BIO CO. LTD.), and 0.55 g of ascorbic acid (L-ascorbic acid (fine powder), manufactured by DSM Japan K.K.), granulating the mixture with 0.85 g of pullulan (Food additive pullulan, manufactured by Hayashibara Co., Ltd.), mixing the obtained granules with 6.36 g of hydroxypropyl cellulose, 3.5 g of sucrose fatty acid ester (RYOTO Sugar Ester S-370FU, manufactured by Mitsubishi Chemical Group Corporation), and 0.40 g of tricalcium phosphate (Food additive tricalcium phosphate, manufactured by Taihei Chemical Industrial Co., Ltd.), and then allowing the mixture to pass through a sieve with a mesh size of 1000 µm.

The obtained mixed powder was compressed into tablets using a fully automatic single-punch tableting machine at a tableting pressure of 10 kN to obtain tablets each having a diameter (ϕ) of 8 mm and a mass of 250 mg. The hardness of the tablet was 94 N and the tablet was disintegrated within 18 minutes.

Formulations and results of the tablets were as shown in Tables 3 and 4. "-" in Table 3 indicates that measurement was not performed (not measured).

**[Table 3]**

| Table 3. Formulations and evaluation results of tablets | | | | | | |
|---|---|---|---|---|---|---|
| | | Component | Example | | | |
| | | | B1 | B2 | B3 | B4 |
| (A) | Lactic acid bacterium | Lactic acid bacterium-sterilized dry powder (mass%) | 2.8 | 2.8 | 2.8 | 2.8 |
| (B1) | Excipient | Hydroxypropyl cellulose (mass%) | 3.5 | 3.5 | 3.5 | 3.5 |
| (B2) | Excipient | Crystalline cellulose (mass%) | 1.6 | 1.6 | 1.6 | 1.6 |
| (C) | Lubricant | Calcium stearate (mass%) | 5.3 | 5.3 | 5.3 | 5.3 |
| | Vitamin | Vitamin D (mass%) | 0.1 | 0.1 | 0.1 | 0.1 |
| (B) | Excipient | Corn protein (mass%) | 0.3 | 0.3 | 0.3 | 0.3 |
| (B) | Excipient | Silicon dioxide (mass%) | 0.7 | 0.7 | 0.7 | 0.7 |
| | Other functional component | 3-hydroxyisovaleric acid calcium (mass%) | 85.8 | 85.8 | 85.8 | 85.8 |
| Total (mass%) | | | 100 | 100 | 100 | 100 |
| (A)/(C) | | | 0.53 | 0.53 | 0.53 | 0.53 |
| (B1)/(C) | | | 0.66 | 0.66 | 0.66 | 0.66 |
| | | Tableting pressure (kN) | 6 | 8 | 10 | 5 |
| Evaluation result | | Tableting failure | Absent | Absent | Absent | Absent |
| | | Diameter (ϕ) (mm) | 9 | 9 | 9 | 9 |
| | | Mass (mg) per tablet (n = 10) | 298.8 | 299.23 | 299.29 | 299.29 |
| | | Tablet hardness (N) (n = 10) | 139.1 | 170.1 | 170.0 | 110.6 |
| | | Disintegration time (within minutes) | - | - | - | - |

**[Table 4]**

| Table 4. Formulations and evaluation results of tablet | | | |
|---|---|---|---|
| | | Component | Example C1 |
| (A) | Lactic acid bacterium | Lactic acid bacterium-sterilized dry powder (mass%) | 3.34 |
| (B1) | Excipient | Hydroxypropyl cellulose (mass%) | 6.36 |
| (B2) | Excipient | β-cyclodextrin (mass%) | 10 |
| (C) | Lubricant | Sucrose fatty acid ester (mass%) | 3.5 |
| | Vitamin | Ascorbic acid (mass%) | 0.55 |
| (B) | Excipient | Pullulan (mass%) | 0.85 |
| (B) | Excipient | Tricalcium phosphate (mass%) | 0.4 |
| | Other functional component | Ornithine hydrochloride (mass%) | 75 |
| Total (mass%) | | | 100 |
| (A)/(C) | | | 0.95 |
| (B1)/(C) | | | 1.82 |
| | | Tableting pressure (kN) | 10 |
| Evaluation result | | Tableting failure | Absent |
| | | Diameter (ϕ) (mm) | 8 |
| | | Mass (mg) per tablet (n = 10) | 250 |
| | | Tablet hardness (N) (n = 10) | 94 |
| | | Disintegration time (within minutes) | 18 |

From the results of Tables 3 and 4, it was confirmed that tableting can be performed without tableting failure in Examples B1 to B4 and C1. Since the tablets of Examples B1 to B4 contain about 2.7×10¹⁰ lactic acid bacteria and 85.5 mass% of 3-hydroxyisovaleric acid calcium per tablet, the tablets have high ingestion efficiency and favorable hardness, so that the tablets are advantageous in that both ingestion efficiency and favorable hardness can be achieved.

Since the tablet of Example C1 contains about 2.8×10¹⁰ lactic acid bacteria and 75 mass% of ornithine per tablet, the tablet has high ingestion efficiency and favorable hardness, so that the tablet is advantageous in that both ingestion efficiency and favorable hardness can be achieved.

Since the tablet of Example C1 had a disintegration time within 18 minutes, the tablet was also advantageous in that absorbability into a living body was high.

### <<Production and Evaluation of Tablet (3)>>

For Examples D1 to D4, tablets were produced and evaluated by the same methods as described in "Production and Evaluation of Tablet (1)" described above, which include (1) Preparation of Lactic Acid Bacterium-Sterilized Dry Powder, (2) Hardness Test, (3) Disintegration Test, and (4) Production and Evaluation Result of Tablet. For Example A1 and Example D2, the degree of browning over time was evaluated.

### Example D1

A mixed powder for tableting was obtained by mixing 15 g of lactic acid bacterium-sterilized dry powder, 4.6 g of hydroxypropyl cellulose (CELNY SSL-SFP, manufactured by Nippon Soda Co., Ltd.), 39 g of crystalline cellulose (Ceolus UF-F702, manufactured by Asahi Kasei Corporation), 4 g of sucrose fatty acid ester (RYOTO Sugar Ester S-370F, manufactured by Mitsubishi Chemical Group Corporation), 13 g of ascorbic acid (Ascorbic acid, manufactured by DSM Japan K.K.), 6 g of tocopherol (Riken Dry E Mix F-20S, manufactured by Riken Vitamin Co., Ltd.), 13.9 g of maltose (SUNMALT-S, manufactured by Hayashibara Co., Ltd.), 1 g of tricalcium phosphate (Tricalcium phosphate, manufactured by Taihei Chemical Industrial Co., Ltd.), 2 g of vitamin A fatty acid ester (Dry Vitamin A, manufactured by Mitsubishi Chemical Group Corporation), 0.3 g of pyridoxine hydrochloride (Vitamin B6 (pyridoxine hydrochloride), manufactured by Watanabe Chemical Co., Ltd.), 0.2 g of riboflavin (Riboflavin, manufactured by KYOWA PHARMA CHEMICAL CO., LTD.), 0.5 g of vitamin D3 (Dry Vitamin D3, manufactured by Mitsubishi Chemical Group Corporation), 0.2 g of thiamine hydrochloride (Thiamine hydrochloride, manufactured by DSM Japan K.K.), and 0.3 g of vitamin B12 (Vitamin B12 0.1% water-soluble powder, manufactured by DSM Japan K.K.), and allowing the mixture to pass through a sieve with a mesh size of 500 µm. The obtained mixed powder was compressed into tablets using a fully automatic single-punch tableting machine (AUTOTAB-535, manufactured by ICHIHASHI SEIKI INDUSTRY CO., LTD.) at a tableting pressure of 15.5 kN to obtain tablets each having a diameter (ϕ) of 9 mm and a mass of 335.5 mg.

It was confirmed that the tablet of Example D1 can be formed into a tablet without tableting failure.

### Example D2

A mixed powder for tableting was obtained by mixing 15 g of a lactic acid bacterium-sterilized dry powder, 4.6 g of hydroxypropyl cellulose, 39 g of crystalline cellulose, 4 g of sucrose fatty acid ester, 13 g of L-ascorbic acid-2-glucoside (Ascofresh, manufactured by Hayashibara Co., Ltd.), 6 g of tocopherol, and 18.4 g of maltose, and allowing the mixture to pass through a sieve with a mesh size of 500 µm. The obtained mixed powder was compressed into tablets using a fully automatic single-punch tableting machine at a tableting pressure of 15.5 kN to obtain tablets each having a diameter (ϕ) of 9 mm and a mass of 335.5 mg.

It was confirmed that the tablet of Example D2 can be formed into a tablet without tableting failure.

For the mixed powders for tableting of Example A1 and Example D2, the degree of browning over time was evaluated.

### Evaluation of Degree of Browning over Time

Example A1 and Example D2 were each allowed to stand still for 7 days under Condition 1 (5°C and about 50% humidity), Condition 2 (60°C and about 50% humidity), or Condition 3 (40°C and about 75% humidity). In Conditions 1 and 2, the mixed powders for tableting were put in a zippered aluminum bag and sealed, and in Condition 3, the mixed powders for tableting were put in a zippered aluminum bag and the bag was opened. Here, the degree of browning of Example D2 over time was evaluated on a six-grade scale while the degree of browning in Condition 1 of Example A1 was "-" (without change) and the degree of browning in Condition 3 of Example A1 was "+++++" (with change). Specifically, the mixed powders for tableting were visually evaluated as "-", "+", "++", "+++", "++++", and "+++++" in order from the mixed powder having no change.

As for the results of the evaluation of the degree of browning over time, in Condition 3 as shown in Fig. 1, the degree of browning of Example A1 was "+++++", whereas the degree of browning of Example D2 was "+++". In Condition 2, the degree of browning of Example A1 was "++", whereas the degree of browning of Example D2 was "-". From these results, it was shown that browning over time can be more suppressed in Example D2 than in Example A1.

### Example D3

A mixed powder for tableting was obtained by mixing 15 g of a lactic acid bacterium-sterilized dry powder, 4.6 g of hydroxypropyl cellulose, 39 g of crystalline cellulose, 4 g of sucrose fatty acid ester, 13 g of rapeseed oil-and-fat processed L-ascorbic acid (VC-80R, manufactured by NOF CORPORATION), 6 g of tocopherol, and 18.4 g of maltose, and allowing the mixture to pass through a sieve with a mesh size of 500 µm. The obtained mixed powder was compressed into tablets using a fully automatic single-punch tableting machine at a tableting pressure of 15.5 kN to obtain tablets each having a diameter (ϕ) of 9 mm and a mass of 335.5 mg.

It was confirmed that the tablet of Example D3 can be formed into a tablet without tableting failure.

### Example D4

A mixed powder for tableting was obtained by mixing 15 g of a lactic acid bacterium-sterilized dry powder, 4.6 g of hydroxypropyl cellulose, 39 g of crystalline cellulose, 4 g of sucrose fatty acid ester, 13 g of corn starch processed L-ascorbic acid (Vitamin C granules-97, manufactured by NOF CORPORATION), 6 g of tocopherol, and 8.4 g of maltose, and allowing the mixture to pass through a sieve with a mesh size of 500 µm. The obtained mixed powder was compressed into tablets using a fully automatic single-punch tableting machine at a tableting pressure of 15.5 kN to obtain tablets each having a diameter (ϕ) of 9 mm and a mass of 335.5 mg.

It was confirmed that the tablet of Example D4 can be formed into a tablet without tableting failure.

## Claims

1. A lactic acid bacterium-containing tablet comprising: (A) a lactic acid bacterium; (B) an excipient; and (C) a lubricant, wherein a content of the (C) in the tablet is less than 10 mass%, wherein a mass per tablet is 200 mg or more.

2. The lactic acid bacterium-containing tablet according to claim 1, wherein the (B) contains at least (B1) hydroxypropyl cellulose.

3. The lactic acid bacterium-containing tablet according to claim 1 or 2, wherein the (B) contains at least one or both of (B2) crystalline cellulose and β-cyclodextrin.

4. The lactic acid bacterium-containing tablet according to claim 2, wherein a content of the (B1) in the tablet is 1 to 20 mass%.

5. The lactic acid bacterium-containing tablet according to claim 2, wherein a ratio of the content (mass%) of the (B1) to the content (mass%) of the (C), expressed as (B1)/(C), is 0.5 to 6.

6. The lactic acid bacterium-containing tablet according to claim 1, wherein the (B) further contains maltose.

7. The lactic acid bacterium-containing tablet according to claim 1, wherein a ratio of a content (mass%) of the (A) to the content (mass%) of the (C), expressed as (A)/(C), is 0.5 to 7.5.

8. The lactic acid bacterium-containing tablet according to claim 1, wherein the content of the (A) in the tablet is 5 to 30 mass%.

9. The lactic acid bacterium-containing tablet according to claim 1, wherein the (A) is a lactic acid bacterium-sterilized dry powder.

10. The lactic acid bacterium-containing tablet according to claim 1, wherein the lactic acid bacterium of the (A) is a bacterium of the genus *Lactococcus.*

11. The lactic acid bacterium-containing tablet according to claim 10, wherein the bacterium of the genus *Lactococcus* is *Lactococcus lactis* subsp. *lactis* JCM5805.

12. The lactic acid bacterium-containing tablet according to claim 1, wherein the (C) is a fatty acid ester.

13. The lactic acid bacterium-containing tablet according to claim 1, further comprising one or more selected from the group consisting of vitamin, 3-hydroxyisovaleric acid, 3-hydroxyisovaleric acid calcium, ornithine, and ornithine hydrochloride.

14. The lactic acid bacterium-containing tablet according to claim 1, which is a food product.

15. The lactic acid bacterium-containing tablet according to claim 1, wherein a hardness of the tablet is 50 N to 200 N.

16. The lactic acid bacterium-containing tablet according to claim 13, wherein the vitamin is L-ascorbic acid-2-glucoside.

17. The lactic acid bacterium-containing tablet according to claim 1, wherein the content of the (C) in the tablet is 3 mass% or more.
